# EUROPEAN PATENT SPECIFICATION

(11) **EP 3 431 586 B1**
(45) Date of publication and mention of the grant of the patent: **10.07.2024**
(21) Application number: 17759829.9
(22) Date of filing: 24.02.2017
(51) Int. Cl.: C12N 5/10

(54) **HIGHLY FUNCTIONAL LIVER CELLS, AND USE THEREOF**
HOCHFUNKTIONALE LEBERZELLEN UND VERWENDUNG DAVON
CELLULES HÉPATIQUES HAUTEMENT FONCTIONNELLES ET UTILISATION CORRESPONDANTE

(30) Priority: 29.02.2016 JP 2016037518
(43) Date of publication of application: 23.01.2019
(73) Proprietor: Yonemitsu, Yoshikazu, Fukuoka-shi, Fukuoka 810-0023 (JP); GAIA BioMedicine Inc., Fukuoka 810-0041 (JP)
(72) Inventor: YONEMITSU, Yoshikazu, Fukuoka-shi Fukuoka 810-0023 (JP); HARADA, Yui, Fukuoka-shi Fukuoka 812-8581 (JP); SAITO, Satoru, Fukuoka 819-0395 (JP)
(74) Representative: Vossius & Partner Patentanwälte Rechtsanwälte mbB
(86) International application number: PCT/JP2017/007025
(87) International publication number: WO 2017/150363

(56) References cited:
- WO-A1-2011/016485
- WO-A1-2012/105505
- JP-A- 2016 026 490
- US-A1- 2009 221 068
- R. ZHANG ET AL: "Identification of Proliferating Human Hepatic Cells From Human Induced Pluripotent Stem Cells", TRANSPLANTATION PROCEEDINGS, vol. 46, no. 4, 1 May 2014 (2014-05-01), ORLANDO, FL; US, pages 1201 - 1204, XP055470307, ISSN: 0041-1345, DOI: 10.1016/j.transproceed.2013.12.021
- JING SHAN ET AL: "Identification of small molecules for human hepatocyte expansion and iPS differentiation", NATURE CHEMICAL BIOLOGY, vol. 9, no. 8, 1 January 2013 (2013-01-01), Basingstoke, pages 514 - 520, XP055576016, ISSN: 1552-4450, DOI: 10.1038/nchembio.1270
- SHAN, JING ET AL.: "Identification of small molecules for human hepatocyte expansion and iPS differentiation", NAT. CHEM.BIOL., vol. 9, no. 8, 2013, pages 514 - 520, XP055576016, DOI: 10.1038/nchembio.1270
- TOMIZAWA, MINORU ET AL.: "An Optimal Medium Supplementation Regimen for Initiation of Hepatocyte Differentiation in Human Induced Pluripotent Stem Cells", JOURNAL OF CELLULAR BIOCHEMISTRY, vol. 116, no. 8, 1 August 2015 (2015-08-01), pages 1479 - 1489, XP055576021, DOI: 10.1002/jcb.25139
- TAKEBE, TAKANORI ET AL.: "Vascularized and functional human liver from an iPSC-derived organ bud transplant", NATURE, vol. 499, 2013, pages 481 - 484, XP055533326
- AMIMOTO, NAOKI ET AL.: "An evaluation of the utility of the hepatic differentiation method using hollow fiber/organoid culture for the development of a hybrid artificial liver device", BIOCHEMICAL ENGINEERING JOURNAL, vol. 56, 2011, pages 69 - 74, XP055540471
- AMIMOTO, NAOKI ET AL.: "Hepatic Differentiation of Mouse Embryonic Stem Cells and Induced Pluripotent Stem Cells During Organoid Formation in Hollow Fibers", TISSUE ENGINEERING, vol. 17, no. 15, 2011, pages 2071 - 2078, XP055540473
- SOTO-GUTIERREZ, ALEJANDRO ET AL.: "Reversal of mouse hepatic failure using an implanted liver-assist device containing ES cell -derived hepatocytes", NATURE BIOTECHNOLOGY, vol. 24, no. 11, 2006, pages 1412 - 1419, XP008127326
- NAOYA KOBAYASHI ET AL.: "Bio Jinko Kanzo", THE JAPANESE JOURNAL OF ARTIFICIAL ORGANS, vol. 38, no. 3, 2009, pages 145 - 151, XP055576054, DOI: 10.11392/jsao.38.145
- NAOYA KOBAYASHI ET AL.: "Hito Haisei Kansaibo no Kan Saibo Bunka to Bio Jinko Kanzo System eno Oyo", GENOMU IGAKU = GENOME MEDICINE, vol. 6, no. 3, 2006, pages 269 - 275, XP009512396, ISSN: 1346-4671

## Description

### Technical Field

The present invention relates to a highly functional hepatocyte induced from an iPS cell, and use thereof.

### Background Art

Even in these days with advanced medical techniques including those for acute stage management, there are still never-ceasing deaths by liver failure in Japan. In particular, fulminant hepatitis, of which mortality rate is as extremely high as 70 to 90%, is a disease that gives bad prognosis. Although liver transplantation provides the highest curative effect as the treatment of liver failure, there are many cases where liver transplantation cannot be performed, because of shortage of donors or drastic progress of acute hepatic failure.

Hybrid artificial liver support systems (HALSSs) are expected as systems used for assisting the functions of the liver in the period before liver transplantation or until regeneration of the patient's own liver. Although primarily cultured swine hepatocytes are preferred as hepatocytes used for the hybrid artificial livers because of favorable growth thereof, it is concerned that reactivation of endogenous retrovirus cannot be completely denied. Furthermore, hepatocytes extracted from brain-dead donors' livers are limited in obtainable quantity. Therefore, human hepatocytes that can be proliferated in vitro are required.

Several methods are known for inducing hepatocytes by differentiation from pluripotent hepatocytes. For example, Patent document 1 discloses a method for differentiating an embryonic stem cell to induce a hepatocyte, wherein embryonic stem cells are cultured in the presence of deletion type hepatocyte growth factor, bFGF, DMSO, and dexamethasone. Since it is known that cells can be placed under conditions closer to the environment in the living body to improve cell functions by three-dimensionally culturing the cells on a scaffold, this patent document proposes to place the ES cells in a reactor after the embryoid body formation, and perform three dimensional induction by differentiation with dHGF, and describes that the ammonia-metabolizing ability, which is the highly developed function specific to the hepatocytes, and so forth are thereby markedly enhanced compared with those obtainable by plate culture. Patent document 2 discloses a method for preparing a liver tissue or organ, which comprises the subclone separation step of re-cloning embryonic stem cells and separating subclones, the screening step of allowing simultaneous formation of embryoid bodies derived from subclones among the aforementioned subclones under the same conditions, then performing adhesion culture of the embryoid bodies, and selecting a subclone that gives a number of embryoid bodies that are pulsing at the point 48 hours after the start of the culture not lower than 70% in terms of embryoid body number, and the subclone culture step of culturing the retrieved subclone. Patent document 2 also describes that the obtained cell has morphological and histological characteristics of normal liver cell or organ, specifically, for example, it has the binucleated morphology, it is positive to albumin staining, and it has ammonia decomposition ability, protein (albumin) production ability, and testosterone hydroxylation activity. Patent document 3 discloses a method for inducing differentiation into the liver. This method comprises the following steps: (i) the step of providing a group of artificial pluripotent stem (iPS) cells, (ii) the step of culturing the cells of the group in an endoderm induction medium to form a group of anterior definitive endoderm (ADE) cells (the endoderm induction medium mentioned above mentioned above is a predetermined chemical medium having the following properties, i.e., it has a fibroblast growth factor activity, stimulates the signaling pathway comprising SMAD2 and SMAD3, and the signaling pathway comprising SMAD1, SMAD5, and SMAD9, and inhibits phosphatidylinositol 3-kinase (PI3K) and glycogen synthase kinase 3β (GSK3β)), and (iii) the step of culturing the ADE cells of the aforementioned group in a hepatic induction medium to form a group of hepatic precursor cells (the hepatic induction medium mentioned above is a predetermined chemical medium that stimulates the signaling pathway comprising SMAD2 and SMAD3). Patent document 4 discloses a method for producing a highly functional hepatocyte using a pluripotent stem cell, which comprises obtaining a pluripotent stem cell-derived primitive endoderm from a pluripotent stem cell by a method comprising the steps (A) and (B), obtaining a liver precursor cell from the pluripotent stem cell-derived primitive endoderm by a method comprising the step (C), and obtaining a highly functional hepatocyte from the liver precursor cell by a method comprising the step (D): (A) the step of performing culture in a serum-free and feeder-free environment, (B) the step of performing culture in the presence of albumin and at least one kind of cytokine, (C) the step of performing culture in the presence of SHH or SHH agonist, and at least one kind of cytokine, and (D) the step of allowing maturation by performing culture in the presence of at least one kind of cytokine.

Patent document 4 also describes that, in a preferred embodiment, an ES cell or iPS cell is used as the pluripotent stem cell, and more preferably, an iPS cell established by using s Sendai virus vector is used as the iPS cell.

### Prior Art References

### Patent documents

Patent document 1: International Patent Publication WO2006/082890 (Japanese Patent No. 4892740)
Patent document 2: Japanese Patent Unexamined Publication (KOKAI) No. 2007-14273
Patent document 3: International Patent Publication WO2012/025725 (Japanese Patent Unexamined Publication (KOHYO) No. 2013-535980)
Patent document 4: International Patent Publication WO2012/105505

### Disclosure of the Invention

### Object to be Achieved by the Invention

Although hybrid artificial livers are expected to serve as a bridge to liver transplantation or regeneration of patient's own liver, about 1 × 10¹⁰ to 10¹¹ hepatocytes are required per one artificial liver module. The important functions such as ammonia-metabolizing ability of the artificial hepatocytes examined so far to date have not been clarified, or it cannot be said that the functions of such hepatocytes are sufficient. The inventors of the present invention conducted various researches for obtaining highly functional hepatocytes that are theoretically able to infinitely proliferate from human iPS cells, and as a result, accomplished the present invention.

The present invention relates to the embodiments as characterized in the claims. Thus, it relates to the following items:
1. A method for preparing an artificial hepatocyte comprising the following steps:
   the differentiation induction step 1 of culturing an iPS cell in a differentiation induction medium I containing activin A,
   the differentiation induction step 2 of culturing a cell obtained in the differentiation induction step 1 in a differentiation induction medium II containing bone morphogenetic protein 4 (BMP4) and fibroblast growth factor 2 (FGF2); and
   the differentiation induction step 3 of culturing a cell obtained in the differentiation induction step 2 in a differentiation induction medium III containing hepatocyte growth factor (HGF), oncostatin M, dexamethasone, and N,N'-(methylenebis)(4,1-phenylene)diacetamide (FH1) and 2-([N-(5-chloro-2-methylphenyl)(methylsulfonamido)-N-(2,6-difluorophenyl)acetamide (FPH1) to obtain an artificial hepatocyte.
2. The preparation method according to item 1, wherein the iPS cell has been produced by using a Sendai virus vector.
3. The preparation method according to item 1 or 2, wherein the differentiation induction steps 1 to 3 are performed in a serum-free and feeder-free environment.
4. The preparation method according to any one of items 1 to 3, wherein the differentiation induction step 1 and the differentiation induction step 2 are performed for 2 to 14 days in total, and the differentiation induction step 3 is performed for 2 to 14 days.

### Effect of the Invention

According to the present invention, a highly functional hepatocyte can be induced from an iPS cell by differentiation. An artificial liver tissue produced by the induction method, and a hybrid artificial liver using the tissue is also disclosed.

### Brief Description of the Drawings

[Fig. 1] Cord blood CD34 positive-cell-derived iPS cells (CiPS)
[Fig. 2] Cord blood CD34 positive-cell-derived iPS cells (CiPS)
[Fig. 3] Confirmation of elimination of the rSeV vector from CiPS
[Fig. 4] Highly functional hepatocytes induced from optimized iPS cells
[Fig.5] Confirmation of ammonia metabolism amount. It was found that use of the compounds (FH1, FPH1) provides higher metabolism-enhancing effect compared with use of feeder cells (HUVECs, MSCs).
[Fig. 6] Confirmation of ammonia metabolism amount. It was found that the obtained iPS-HEP cell has ammonia-metabolizing function comparable to the metabolizing ability of the primarily cultured hepatocyte (160 to 200 µg/dl/24h) depending on the culture conditions.
[Fig. 7] Confirmation of hepatocyte markers (RT-PCR)
[Fig. 8] Confirmation of hepatocyte markers (RT-PCR)
[Fig. 9] Confirmation of hepatocyte markers (RT-PCR)

### Modes for Carrying out the Invention

Numerical value ranges indicated as "X to Y" include the values of X and Y as the maximum and minimum values, unless especially indicated. The expression "A and/or B" means at least one of A and B, unless especially indicated.

### <Artificial hepatocyte>

There is disclosed an artificial hepatocyte having a high ammonia-metabolizing function and able to constitute a mesh structure.

### [Mesh structure]

The expression "able to constitute a mesh structure" used for the characteristics of the cell means that when the objective cell is cultured under appropriate conditions, a predetermined mesh structure is constituted. The mesh structure is constituted if the cells are inoculated in a culture vessel, which may be coated with an extracellular matrix (ECM) such as Matrigel (registered trademark), hyaluronic acid, heparin, fibronectin, laminin, vitronectin, or other ECM as required, at an appropriate density, and cultured for several days using an appropriate medium. The mesh structure comprises string portions having a thickness corresponding to a width of at least one cell to 1000 µm (more specifically, a width of several cells to 500 µm, further specifically 20 to 250 µm), and voids having a circular shape, elliptical shape, or the like. The diameters of the voids are typically 100 to 2000 µm, more specifically 200 to 1000 µm, further specifically 300 to 1000 µm. The mesh structure may have a thickness of 10 to 60 µm corresponding to the width of 1 to 3 cells.

### [Ammonia-metabolizing ability (function)]

The term ammonia-metabolizing function referred to in the present disclosure means ammonia-metabolizing function observed when the objective cell is cultured under appropriate conditions, unless especially indicated. Methods for measuring ammonia-metabolizing function are well known to those skilled in the art. It is preferable to perform adhesion culture using a culture vessel coated with EMC as required and the compound mentioned later (FH1) without using a feeder cell (HUVECs, MSCs, and the like). As for more detailed conditions, the methods described in the section of Examples of the present specification can be referred to.

The artificial hepatocyte obtained by the present invention has a high ammonia-metabolizing function. Specifically, the artificial hepatocyte as disclosed herein has an ammonia-metabolizing ability not lower than 100 µg/dl/24h, preferably not lower than 120 µg/dl/24h, more preferably that comparable to the metabolizing ability of the primarily cultured hepatocyte (160 to 200 µg/dl/24h), i.e., 160 µg/dl/24h, still more preferably 180 µg/dl/24h.

### [Characteristics of culture]

### Culture in serum-free and/or feeder-free environment

As the medium for culturing artificial hepatocyte of the present invention, the various existing media developed for culturing hepatocytes can be used. Examples of usable medium include the differentiation induction medium III mentioned later, HCM^{™} BulletKit^{™} Medium (Lonza Walkersville, Inc), and HBM^{™} Basal Medium (Lonza Walkersville, Inc).

The artificial hepatocyte of the present invention can be cultured in a serum-free and/or feeder-free environment, preferably a serum-free and feeder-free environment. To culture in a serum-free and feeder-free environment means to perform culture by using a medium containing neither animal serum nor human serum in an environment where any cells other than the objective hepatocyte, such as mouse fetal fibroblast, a feeder cell (human umbilical vein endothelial cell (HUVEC)), or mesenchymal stem cell (MSC), do not coexist.

### EMC coat

For the culture of the artificial hepatocyte of the present invention, various kinds of existing culture vessels can be used. Although material of the culture vessel can be appropriately chosen by those skilled in the art, it is preferable to use a culture vessel coated with EMC, since such a culture vessel provides favorable growth and maintains high functionality of the hepatocyte. Preferred examples of ECM include Matrigel (registered trademark), hyaluronic acid, heparin, fibronectin, laminin, vitronectin, proteoglycan (chondroitin sulfate proteoglycan, heparan sulphate proteoglycan, keratan sulfate proteoglycan, dermatan sulfate proteoglycan), various collagens, gelatin, tenascin, entactin, elastin, fibrillin, and so forth.

### Maintenance and subculture

It has been confirmed that the artificial hepatocyte of the present invention can be continuously cultured for about ten days by using the differentiation induction medium III mentioned later. It is considered that the hepatocyte can be subcultured and proliferated by inoculating it at an appropriate density. The appropriate density is, for example, such a density that the artificial hepatocytes exactly contact with one another. A density lower than such a density provides bad proliferation, and a density higher than such a density may induce marked cell death in a region where three-dimensional construction is observed.

### <Method for producing artificial hepatocyte>

The artificial hepatocyte of the present invention can be produced by the method comprising the following steps:
the differentiation induction step 1 of culturing an iPS cell in the differentiation induction medium I containing activin A,
the differentiation induction step 2 of culturing a cell obtained in the differentiation induction step 1 in the differentiation induction medium II containing bone morphogenetic protein 4 (BMP4) and fibroblast growth factor 2 (FGF2); and
the differentiation induction step 3 of culturing a cell obtained in the differentiation induction step 2 in the differentiation induction medium III containing hepatocyte growth factor (HGF), oncostatin M, dexamethasone, and a compound having N-phenyl-2-(N-phenylmethylsulfonamido)acetamide structure to obtain an artificial hepatocyte..

### Differentiation induction step 1:

In the differentiation induction step 1, an iPS cell is cultured in the differentiation induction medium I containing at least activin A.

### iPS Cell

In the present invention, an iPS cell derived from human or nonhuman animal is used as the starting cell. Methods for obtaining and establishing an iPS cell are well known to known those skilled in the art. Human iPS cells can be purchased or provided from Institute of Physical and Chemical Research (Riken) BioResource Research Center, Kyoto University, or National Center for Child Health and Development. Examples of iPS cells that are available and can be used for the present invention include, for example, those of the human artificial pluripotent stem (iPS) cell strain HiPS-RIKEN-1A (Institute of Physical and Chemical Research, HPS0003), which was established by introducing four factors (Oct3/4, Sox2, Klf4, and c-Myc) into an umbilical cord-derived fibroblast (RCB0436 HUC-F2) using a retrovirus vector, human iPS cell strain HiPS-RIKEN-2A (Institute of Physical and Chemical Research, HPS0009), which was established by introducing four factors (Oct3/4, Sox2, Klf4, and c-Myc) into an umbilical cord-derived fibroblast (RCB0197 HUC-Fm) using a retrovirus vector, human iPS cell strain HiPS-RIKEN-12A (Institute of Physical and Chemical Research, HPS0029), which can be obtained by introducing three factors (Oct3/4, Sox2, and Klf4) into an umbilical cord-derived fibroblast using a retrovirus vector, human iPS cell strain Nips-B2 (Institute of Physical and Chemical Research, HPS0223), which was established by introducing four factors (Oct3/4, Sox2, Klf4, and c-Myc) using a Sendai virus vector, and so forth.

### Use of Sendai virus vector

When an iPS cell is established, it is preferable to use a Sendai virus vector as the vector for introducing the factors. Retrovirus vectors enter into cell nuclei, and induce gene expression as DNA, and therefore it is concerned that the vectors may positively enter into patient's chromosomes, or cause genetic recombination with a chromosomal DNA, when the obtained iPS cell is used for the patient, even though such events may very rarely occur. On the other hand, Sendai virus vectors do not enter into cell nuclei, but replicate genomes thereof within the cytoplasm, and produce a lot of proteins. These genomes are made of RNA, and they materially differ from DNAs of the patient's chromosomes. Therefore, it is considered that there is theoretically no risk of modification of chromosomes of the patient in the nucleus by a Sendai virus vector.

Methods for establishing an iPS cell using a Sendai virus (SeV) vector are well known to those skilled in the art, and it can be established by, for example, culturing a commercial human fibroblast or the like in a medium containing a Sendai virus vector carrying a reprogramming factor expression unit. Examples of such a Sendai virus vector carrying a reprogramming factor expression unit include, for example, CytoTune-iPS (DNAVEC Corporation), and so forth.

### Differentiation induction medium I

The differentiation induction medium I used in this step contains a cytokine such as Wnt3A or activin A. It is preferred that at least activin A is contained. As for the amount of the cytokine, amount of Wnt3A is, for example, about 10 to 50 ng/mL, preferably about 25 ng/ml, and amount of activin A is, for example, about 10 to 100 ng/ml, preferably about 100 ng/ml.

The differentiation induction medium I is free from serum, and for example, a medium having a basic composition such as RPMI1640 to which the aforementioned cytokine and serum substitute are added can be used. Examples of the serum substitute include B27 (registered trademark, Life Technologies), KnockOut (registered trademark) Serum Replacement (Life Technologies), and so forth. A specific example of the composition of the differentiation induction medium I is that mentioned in the section of Examples in this specification. As for the composition of B27, G.J. Brewer et al., Optimized Survival of Hippocampal Neurons in B27-Supplemented Neurobasal™, a New Serum-free Medium Combination, Journal of Neuroscience Research 35567476 (1993) can be referred to.

This step is performed by culturing iPS cells at 37°C for several days in a 5% CO₂ incubator using the differentiation induction medium I. The culture period of the differentiation induction step 1 is specifically 4 to 10 days, preferably 6 to 8 days, more preferably 7 days. The differentiation induction step 1 can be performed until the cells come to exhibit morphological characteristics of endomere.

### Differentiation induction step 2

In the differentiation induction step 2, the cells obtained by the differentiation induction step 1 are cultured in the differentiation induction medium II.

### Differentiation induction medium II

The differentiation induction medium II used in this step contains various cytokines, for example, fibroblast growth factor 2 (FGF2), bone morphogenetic protein 4 (BMP4), and hepatocyte growth factor (HGF). As for the amounts of the cytokines, amounts of FGF2, HGF, and BMP4 are, for example, 5 to 50 ng/ml, 5 to 50 ng/ml, and 5 to 50 ng/ml, respectively, preferably about 10 ng/ml, about 20 ng/ml, and about 20 ng/ml, respectively. The differentiation induction medium II contains at least the bone morphogenetic protein 4 (BMP4) and the fibroblast growth factor 2 (FGF2). The differentiation induction medium II can also be serum-free, and for example, a medium having a basic composition such as RPMI1640 to which the aforementioned cytokines and serum substitute are added can be used. A specific example of the composition of the differentiation induction medium II is that mentioned in the section of Examples in this specification.

This step can be performed by culturing the cells obtained in the step 1 at 37°C in a 5% CO₂ incubator using the differentiation induction medium II. The culture period of the differentiation induction step 2 is specifically 1 to 6 days, preferably 2 to 5 days, more preferably 3 to 4 days.

### Differentiation induction step 3

This step is a step of culturing the cells obtained in the differentiation induction step 2 in the differentiation induction medium III to obtain artificial hepatocytes.

### Differentiation induction medium III

Examples of the cytokine and hormone used in this step include oncostatin M (OSM), dexamethasone, and so forth. Amounts to be used of OSM and dexamethasone are, for example, 10 to 50 ng/ml, and 0.05 to 0.5 µM, respectively, preferably about 25 ng/ml, and about 0.1 µM, respectively. The differentiation induction medium III contains at least hepatocyte growth factor (HGF), oncostatin M, dexamethasone, and N,N'-(methylenebis)(4,1-phenylene)diacetamide (FH1) and/or 2-(N-(5-chloro-2-methylphenyl)(methylsulfonamido)-N-(2,6-difluorophenyl)acetamide (FPH1). Specific examples of the composition of the differentiation induction medium

### II are those mentioned in the section of Examples in this specification.

### FH1 and FPH1

The differentiation induction medium III contains N,N'-(methylenebis)(4,1-phenylene)diacetamide (FH1) and/or 2-(N-(5-chloro-2-methylphenyl)(methylsulfonamido)-N-(2,6-difluorophenyl)acetamide (FPH1) represented by the following formulas.

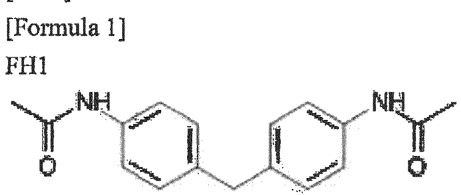

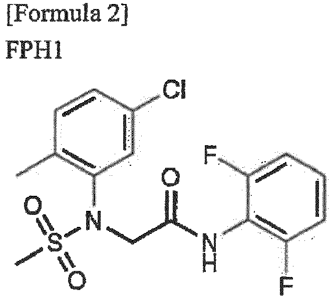

The differentiation induction medium III contains FH1 and FPH1.

This step can be performed by culturing the cells obtained in the differentiation induction step 2 in the differentiation induction medium III for about several days to several weeks. In this culture period, it is preferable to exchange the medium at a frequency of at least every 3 days, preferably everyday.

### Other conditions

All of the differentiation induction steps 1 to 3 can be performed under a serum-free and/or feeder-free environment, preferably serum-free and feeder-free environment. They can also be performed by using a culture vessel coated with ECM, as required.

### Confirmation of hepatocytes

It is known that hepatic parenchymal cells embryologically appear following the appearance of the cardiac muscle cells in the embryoid body. It is experimentally known that expression of hepatic parenchymal cells is observed near the cardiac muscle cells differentiated from the embryoid bodies.

As for the method for confirming the differentiation of the cells undergone the differentiation induction steps 1 to 3 into hepatocytes, the differentiation can be confirmed on the basis of expression of a hepatocyte (fetal hepatocyte)-specific marker gene or expression of a hepatocyte-specific marker protein such as transthyretin (TTR), α-fetoprotein (AFP) fetal hepatocyte, a1-antitrypsin (AAT), tyrosine aminotransferase (TAT), tryptophan oxygenase (TO), tyrosine aminotransferase, asialoglycoprotein receptor (ASGR), and albumin, or by immunostaining using an anti-albumin antibody. Further, since many binucleated cells are observed among mouse hepatocytes, the differentiation can be confirmed by confirming morphology of the cell nuclei.

The confirmation is preferably performed on the basis of criteria that they can constitute a mesh structure, and they have a high ammonia-metabolizing ability, as described in the section of the artificial hepatocyte mentioned above.

### <Others>

### (Hybrid artificial liver)

There is also disclosed a hybrid artificial liver and a method for producing the same. It is preferred that the hybrid artificial liver uses at least 10⁹ of the artificial hepatocytes as disclosed herein.

Hybrid artificial livers are classified into three forms, i.e., those equipped outside the body and connected to a blood vessel, those retained inside the body and connected to a blood vessel, and those retained in the abdominal cavity without connecting to a blood vessel. Since the hybrid artificial liver as disclosed herein uses hepatocytes derived from iPS cells, it is considered that it is preferably of the extracorporeal type in order to avoid the risk concerning cell transfer, and so forth.

In the development of a hybrid artificial liver, design and development of a reactor are also important elements. As bioreactors, there are known those of various types, such as HepatAssist (Hui T, Rozga J, Demetriou AA, J. Hepatobiliary Pancreat. Surg., 2001, 8:1-15), which is for hybrid artificial liver treatment using swine hepatocytes, and MELS (Modular Extracorporeal Liver System) using swine hepatocytes. These reactors can also be used in the context of the present disclosure. Hepatocytes in a floating state tend not to show sufficient differentiation function, collide with surrounding cells, and easily receive stress stimuli. Further, it is considered that a reactor comprising a scaffold of hollow fibers and nonwoven fabric etc. is preferred so that a scaffold can be provided to the hepatocytes, since it has been found that the artificial hepatocytes as disclosed herein provide higher ammonia-metabolizing ability when they are cultured as adhesion culture according to the researches conducted by the inventors of the present invention.

Any hollow fiber membrane can be used so long as the cells can adhere to the membrane surface, and substance exchange is not disturbed, and specifically, commercial products conventionally used for medical uses, for example, polysulfone membrane, saponified ethylene-vinyl acetate random copolymer membrane (for example, Eval (trade name), Kuraray Medical Inc., etc.), and so forth are preferred. As for pore sizes of commercial hollow fiber membranes, there are those having various pore sizes depending on uses thereof, such as dialysis membrane (5 nm or smaller), plasma segregation membrane (20 to 30 nm), and plasma skimming membrane (30 to 200 nm). From the viewpoint of substance permeability, a plasma skimming membrane (30 to 200 nm) is preferred. In order to avoid the risk of rejection, the pore size is most preferably 30 to 100 nm, so that immunocompetent cells and immunoglobulin in the blood flowing through the inside of the hollow fibers should not directly contact with the cells filled on the scaffold of nonwoven fabric or the like outside the hollow fibers.

As the nonwoven fabric, those processed and modified so that cells can adhere to them are preferred. Examples of the fibers of the nonwoven fabric include those of polytetrafluoroethylene (PTFE), and so forth. Those consisting of polytetrafluoroethylene (PTFE) treated with polyamino acid urethane (PAU) are particularly preferred, because of ease of processing thereof.

### (Toxicity test)

The artificial hepatocytes obtained according to the present disclosure can be used for a test for evaluating toxicity of drugs. When they are used for a toxicity test, an objective drug is added to the artificial hepatocytes, the cells are cultured, for example, at 37°C in a 5% CO₂ incubator, and the toxicity can be evaluated on the basis of change of ammonia-metabolizing ability with time, or presence or absence, or degree of cell death. Examples of techniques for measuring cell death include detection of fragmentation of chromosomal DNA by electrophoresis, detection of chromosomal DNA-reduced cells using flow cytometry, detection of cells causing apoptosis using annexin V, detection of dead cells using dead cell staining agent such as propidium iodide (PI), ATP assay, MTT assay, intracellular glutathione assay, LDH assay, and so forth.

### (Method for drug screening)

As disclosed herein, the artificial hepatocytes obtained according to the present invention can be used for drug screening. Specific examples of the method for drug screening include, for example, a method of adding a test substance to a culture medium of a tissue comprising the artificial hepatocytes of the present disclosure or artificial hepatocytes, incubating them for a predetermined time, and then analyzing a metabolic product contained in at least either one of the culture medium and the cells constituting the liver tissue or organ, or the like, and so forth.

The test substance is not particularly limited, and can be appropriately chosen depending on the purpose, and examples include, for example, drugs, diagnostic agents, and functional foods relating to liver functions, as well as active ingredients of these. Drug screening can also be performed as screening for ingredients useful for improvement of liver functions, or screening for harmful ingredients that damage the liver. Examples of the screening for ingredients useful for improvement of the liver functions include, for example, searches for a cholesterol decomposition-promoting substance, a cholesterol biosynthesis inhibitor, and so forth.

### Examples

### <Preparation of iPS cells>

### [Preparation of iPS cells]

iPS cells were prepared by the following method.
1. Thaw POIETICS (registered trademark) CORD BLOOD CD34+ Cells (Cat. No. 2C-101A) and POIETICS (registered trademark) Mobilized CD34+ Cells (Cat. No. 2G-101B).
2. Prepare IPS cells by using CytoTune (registered trademark)-IPS 2.0 Sendai Reprogramming Kit according to the protocol attached to the kit.

### [Confirmation of iPS cells]

The following procedure was performed to confirm acquisition of iPS cells.

### Immunostaining

1. Stick 253G1 (obtained from Institute of Physical and Chemical Research (Riken)) and CiPS to slide glass using CytoSpin (registered trademark) (800 rpm, 5 minutes)
2. Perform fixation with 4% paraformaldehyde (Nakalai Tesque) (15 minutes, room temperature)
3. Perform washing 3 times with PBS
4. Perform blocking (0.3% Triton X-100, 1% BSA, 10% AB serum, or Blocking One-Histo (Nakalai Tesque)) (10 to 20 minutes, room temperature)
5. Perform treatment with 0.1% Tween/PBS, 5 minutes, room temperature
6. R&D, Human Pluripotent Stem Cell 3 Color Immunocytochemistry Kit, 10-fold dilution for each, 3 hours, room temperature or o/n, 4°C
7. Perform washing with PBS
8. Perform enclosure with VECTA SHIELD (registered trademark) Hard Set with DAPI
9. Perform observation with the all-in-one fluorescence microscope BZ-9000 (KEYENCE).

### Flow cytometry

1. Separate 253G1 (obtained from Institute of Physical and Chemical Research (Riken)) and CiPS by a treatment with Accutase at 37°C for 5 minutes, and then collect them.
2. Perform staining with SSEA-4 FITC, SSEA-3 PE, and TRA-1-81 PE antibodies (final concentration, 1 µg/ml for each) (4°C, 30 minutes).
3. Perform measurement with FACSCalibur.
4. Perform analysis with CellQuest or FlowJo.

### Morphological observation

During the culture performed as described later, morphology was periodically observed with the all-in-one fluorescence microscope BZ-9000 (KEYENCE).

### Confirmation of elimination of SeV vector (CytoTune iPS2.0)

1. Culture iPS cells on a 12-well plate.
2. Perform washing with PBS.
3. Perform fixation with 1 ml/well of 10% neutral formalin (5 minutes, room temperature).
4. Perform washing with PBS.
5. Add 500 µl of Anti-SeV antibody (diluted 500 times with 0.1% Triton X-100/PBS).
6. Allow reaction at 37°C for 1 hour.
7. Perform washing with PBS.
8. Add 500 µl of DyLight 550-labeled anti-rabbit IgG-antibody (diluted 500 times with 0.1% Triton X-100/PBS).
9. Allow reaction at 37°C for 1 hour.
10. Perform washing with PBS.
11. Perform observation with the all-in-one fluorescence microscope BZ-9000 (KEYENCE).

### Results

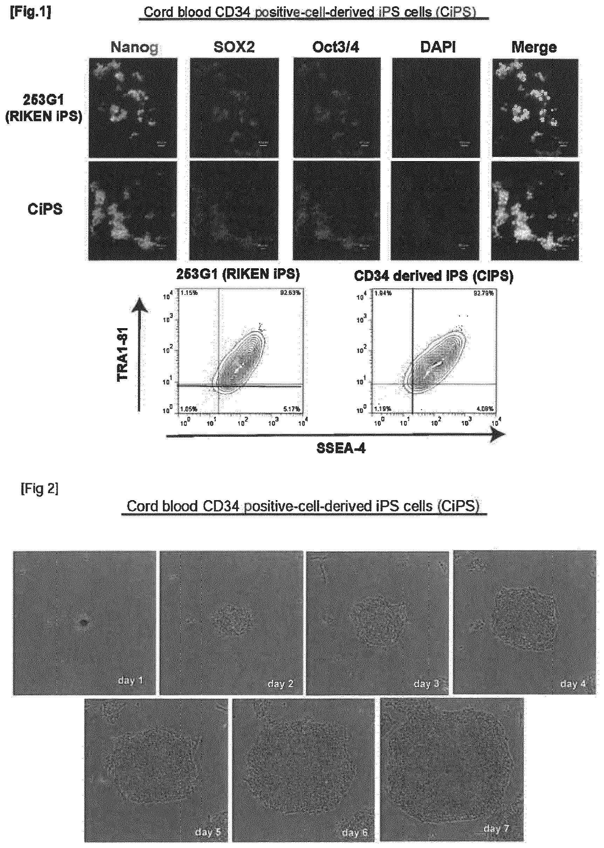

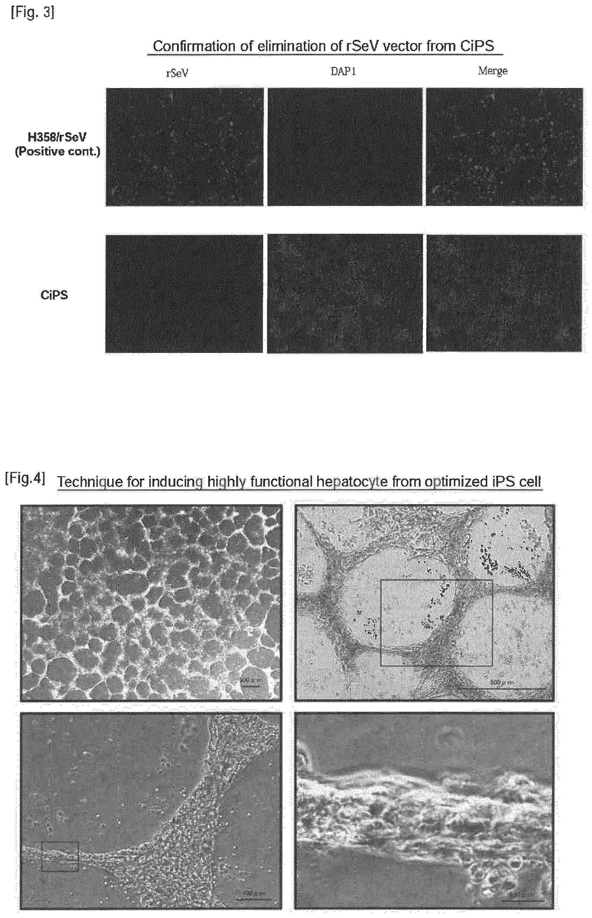

The results of the immunostaining and flow cytometry are shown in Fig. 1, the results of the periodical observation of the cell morphology are shown in Fig. 2, and the results of the confirmation of elimination of the rSeV vector from the CiPS cells are shown in Fig. 3. The CiPS cells were in a feeder-free environment through out the whole process from the preparation thereof to the expansion culture. CiPS from which sReV was eliminated could be obtained by subculture for 3 months from the infection.

### <Preparation of iPS cells for inducing CiPS-Hep>

According to the following method, iPS cells for inducing iPS-derived highly functional hepatocytes (CiPS-Hep) were prepared. In the confirmation of the aforementioned prepared iPS cells such as iPS cell colony observation, the methods described in this section were performed.

### [Preparation of laminin-coated dish]

1. Melt laminin-5¹⁾ (-80°C) at 4°C. Pay attention not to warm it with hand.
2. Put PBS³⁾ into wells of a 6-well plate²⁾ in a volume of 1 ml/well, and spread it over each well.
3. Cool the 6-well plate containing PBS and 200-µL tip at 4°C.
4. Put the melted laminin-5 into the wells containing PBS in a motion of drawing a spiral pattern, and quickly sway the plate about 10 times, so that laminin-5 spreads over the whole well. Since laminin-5 is highly adsorptive, never perform pipetting.
5. Attain coating by incubation at 4°C overnight, or at 37°C for 2 hours or longer. Don't leave the plate at 37°C.
6. When the plate is not used immediately, it can be doubly sealed with Parafilm, and stored at 4°C (1 week).

### [Thawing and culture of frozen cells]

1. Set P2 centrifuge at 20°C.
2. Take out various inhibitors (4 kinds used in the following step 9) from a freezer at - 30°C.
3. Prepare culture medium (henceforth referred to as ReproFF2).

**[Table 1]**

| ReproFF2⁴⁾ | | 500ml | |
|---|---|---|---|
| +FGF2 ⁵⁾ | 1mg/ml | 2.5µl | (Final 5ng/ml) |
| +penicillin/ streptomycin⁶⁾ | | 5ml | (Final 1%) |

4. Put ReproFF2 into 15-ml tubes⁷⁾ in a volume of 9 ml or 4 ml each, and warm it at 37°C.
5. Rinse twice the laminin-coated dish with PBS(-), and put 1 ml each of PBS(-) into the wells.
6. Take out the frozen cells from liquid nitrogen, and immerse them into a water bath at 37°C for 2 minutes to thaw them. When the volume of the frozen cells is small, add an appropriate volume of warmed culture medium to the cells, and perform gentle pipetting to quickly thaw them.
7. Collect the thawed cells, transfer them into a 15-ml tube containing 9 ml of the culture medium, and mix them by gentle inversion of the tube.
8. Centrifuge them under the conditions of 160 g, 5 minutes, and room temperature with acceleration and braking.
9. Separately prepare 4 ml of culture medium + each inhibitor during the centrifugation.

**[Table 2]**

| ReproFF2 | | | 4ml |
|---|---|---|---|
| +Thiazovivin⁸⁾ | 5mM | 4µl | (Final 5µM) |
| +CHIR99021⁹⁾ | 1mM | 4µl | (Final 1µM) |
| +PD0325901¹⁰⁾ | 400µM | 4µl | (Final 0.4µM) |
| +SB431542¹¹⁾ | 2mM | 4µl | (Final 2µM) |

10. Collect the 15-ml tube after centrifugation, and carefully remove the supernatant.
11. Add 1 ml of the inhibitor + culture medium, and gently loosen the cell pellet by pipetting.
12. Remove PBS(-) on the laminin-coated dish, and transfer the cell-suspending culture medium to the well. Wash the 15-ml tube with the remaining inhibitor + culture medium, and transfer it to the well.
13. Sway the dish to uniformly spread the cells, and incubate them at 37°C and 5% CO₂.
14. Gently stir the medium in the well on the next day, and then remove the supernatant.
15. Add 4 ml of culture medium + each inhibitor again, and perform culture at 37°C and 5% CO₂.

### [Medium exchange]

1. Remove the culture supernatant.
2. Add fresh culture medium ReproFF2 in a volume of 4 ml/well.
3. Perform the aforementioned operations every 2 or 3 days (on Monday, Wednesday, and Friday).

### [Subculture of cells]

1. Prepare a laminin-coated dish.
2. Remove the culture supernatant.
3. Add 1 ml of PBS(-) to wash cell surfaces.
4. Remove PBS(-), add ESGRO Complete Accutase¹²) in a volume of 1 ml/well, and perform incubation at 37°C and 5% CO₂ for 5 minutes.
5. Separate the cells not completely separated in the well by pipetting, and collect the cell suspension into a 15-ml tube.
6. Add 1 ml of ReproFF2 to a well to wash the inside of the well, collect the medium into the 15-ml tube, and add further 1 ml of ReproFF2 to the 15-ml tube to obtain a total volume of 3 ml.
7. Sufficiently mix the prepared cell suspension, then dilute it 2-fold with a trypan blue staining solution¹³), and perform cell counting.
8. Put the cell suspension into new 15-ml tubes depending on the number of wells in which subculture is performed, so that a cell density of 5 × 10⁵ cells/well is obtained. Cryopreserve the remaining cells if needed.
9. Centrifuge them under the conditions of 160 g, 5 minutes, and room temperature with acceleration and braking.
10. Prepare 4 ml of the culture medium + each inhibitor during the centrifugation.

**[Table 3]**

| ReproFF2 | | | 4ml |
|---|---|---|---|
| +Thiazovivin | 5mM | 4µl | (Final 5µM) |

11. Remove the supernatant after the centrifugation, loosen the cell pellet by pipetting using the culture medium + inhibitor, and inoculate the cells on each laminin-coated well.
12. Indicate the date, name of the iPS cell, and passage number on the culture plate, and perform culture at 37°C and 5% CO₂. Continue the subculture by performing the subculture operations on Monday, Friday, Wednesday, and Monday again.

### [Cryopreservation of cells]

1. Separate the cells in the same manner as that used in the subculture, and count them.
2. Centrifuge the cell suspension under the conditions of 160 g, 5 minutes, and room temperature with acceleration and braking.
3. Attach a label indicating the date, cell name, and passage number to serum tubes¹⁴⁾ used for freezing.
4. Sufficiently remove the supernatant after centrifugation, and suspend the cells in STEM-CELLBANKER¹⁵⁾ at a density of 1 × 10⁶ cells/500 µl.
5. Put the cell suspension into the labeled serum tubes in a volume of 500 µl/tube.
6. After putting the cell suspension, put the serum tubes into a -80°C deep freezer.
7. Transfer the frozen cells from -80°C to a liquid nitrogen cell preservation system within 1 to 3 days, and store them.

### <Induction of CiPS-Hep>

According to the following method, CiPS cells were differentiated to induce highly functional hepatocytes (CiPS-Hep).

### [Day 1: iPS-derived highly functional hepatocyte induction by differentiation step 1 - medium change to iPS-derived highly functional hepatocyte induction by differentiation medium I (henceforth referred to as medium I)]

1. Prepare a required amount of the medium I (4 ml/well). 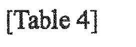 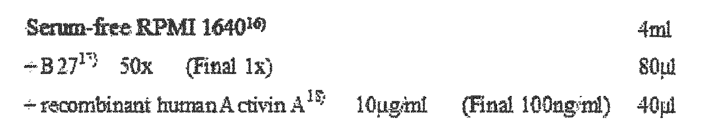
2. Remove the supernatant, and add 1 ml of serum-free RPMI 1640 to wash cell surfaces.
3. Add the medium I in a volume of 4 ml/well.
4. Perform an operation of removing the supernatant, and adding fresh medium I everyday up to Day 6.

### [Day 7: iPS-derived highly functional hepatocyte induction by differentiation step 2change of medium to iPS-derived highly functional hepatocyte induction by differentiation medium II (henceforth referred to as medium II)]

1. Prepare a required amount of medium II (4 ml/well). 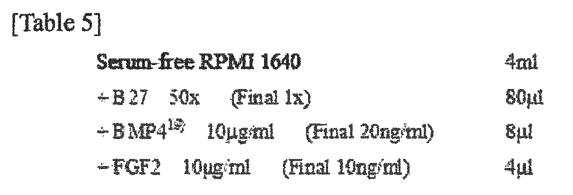
2. Perform gentle pipetting of the culture supernatant, then remove the supernatant, and add the medium II in a volume of 4 ml/well.
3. Perform an operation of removing the supernatant, and adding fresh medium II everyday up to Day 9.

### [Day 10: iPS-derived highly functional hepatocyte induction by differentiation step 3]

1. Matrigel coating of culture plate
   1.1. Thaw 300 µl of BD Matrigel Growth Factor Reduced²⁰⁾ at 4°C.
   1.2. Cool 24-well plate dish²¹⁾ and KnockOut-DMEM²²⁾ at 4°C.
   1.3. When Matrigel is thawed, add the same volume of KnockOut-DMEM, and sufficiently mix them by pipetting.
   1.4. Put the diluted Matrigel solution into wells of the 24-well plate dish in a volume of 200 µl per one well, and spread it over the whole well.
   1.5. Leave the 24-well plate dish standing at ordinary temperature for 3 hours or longer to attain coating.
2. Subculture of iPS cell-derived highly functional hepatocytes
   2.1. Prepare iPS-derived highly functional hepatocyte induction by differentiation medium III (henceforth referred to as medium III) in a volume of 1 ml/well. 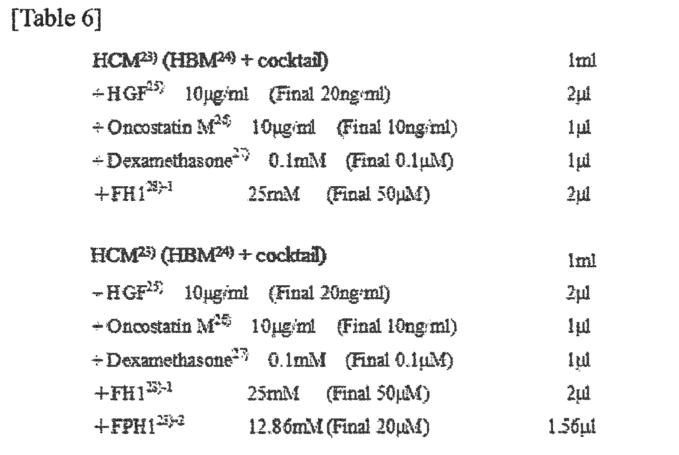
   2.2. Remove the culture supernatant, and wash the cell surfaces with 1 ml of PBS(-).
   2.3. Add 1 ml of ESGRO Complete Accutase, and carry out incubation at 37°C for 5 minutes.
   2.4. Deliberately carry out pipetting to separate the cells, and collect the cell suspension into a 15-ml tube.
   2.5. Wash the inside of the well with 1 ml of HCM, collect the solution in the 15-ml tube, and further add 1 ml of HCM to the 15-ml tube to obtain a total volume of 3 ml.
   2.6. Sufficiently stir the cell suspension, and carry out cell counting using trypan blue diluted 2-fold.
   2.7. Put the cell suspension into new 15-ml tubes depending on the number of wells for subculture so that a density of 1.0 × 10⁶ cells/well is obtained.
   2.8. Carry out centrifugation at 160 g and room temperature for 5 minutes.
   2.9. During the centrifugation, add 1 ml of PBS(-) to the Matrigel-coated well to carry out washing, and remove the supernatant. Repeat this washing operation twice, and add 1 ml of PBS(-) to the well.
   2.10. After the centrifugation, remove the supernatant and also the supernatant in the Matrigel-coated well when adhesion culture is performed, then loosen the cell pellet with the medium III, and inoculate the cells into the Matrigel-coated well.
      When floating culture is performed, loosen the cell pellet by pipetting using the medium III, and inoculate the cells onto an MFC treatment plate (MD6 with Lid Low-Cell Binding, Nalge Nunc International, Japan).
      When HUVEC and MSC are used as feeder cells, perform co-culture as required as adhesion culture (refer to Non-patent document 1 cited below).
   2.11. Indicate the date, name of the cells, etc. on the culture plate, and carry out culture in an incubator at 37°C and 5% CO₂.
   2.12. Carry out an operation of removing the supernatant, and adding fresh medium III on every other day up to Day 17.

### [Day 17: Completion of CiPS-Hep -> ammonia metabolism test]

1. Prepare HBM (cocktail) + aqueous ammonia (henceforth referred to as ammonia medium) in a volume of 1 ml/well × 2.

**[Table 7]**

| | |
|---|---|
| HBM | 1ml |
| **Aqueous ammonia²⁹⁾ (1M)** | 2µl (2mM) |

2. Remove the culture supernatant, and wash the cell surfaces once with 1 ml of HBM, and once with ammonia HBM.
3. Remove the supernatant, and add 1 ml/well of the ammonia medium.
4. Put the ammonia medium or HBM (1 ml each) into empty wells and used as positive control and negative control.
5. Carry out culture at 37°C and 5% CO₂ for 24 hours.

### [Day 18: Collection of sample for ammonia metabolism test]

1. Collect the supernatant into an Eppendorf tube, and carry out centrifugation at 500 g for 5 minute.
2. Collect the supernatant into three of new Eppendorf tubes in a volume of 300 µl each, and freeze it at -80°C.
3. Wash the well with PBS, add 1 ml of trypsin-EDTA³⁰⁾, and carry out incubation at 37°C for 5 minutes.
4. Deliberately carry out pipetting to separate the cells, and put the cell suspension into an FACS tube³¹⁾.
5. Carry out washing with 1 ml of HBM, and make the total volume 2 ml.
6. Perform counting using trypan blue.

### <Confirmation of iPS-HEP cell>

Acquisition of iPS cells was confirmed by carrying out the following procedure.

### Ammonia Test Wako³²⁾

1. Dissolve the supernatant.
2. Carry out centrifugation at 8000 rpm and 4°C for 5 minutes.
3. Prepare diluted ammonia solutions. Not use the standard solution and the standard diluted solutions contained in the kit. Add 40 µl of an ammonium ion standard solution (1000 mg/L) to 960 µl of HBM (additive-free), and vortex them to prepare a standard solution.

**[Table 8]**

| | |
|---|---|
| 0 µg/dl: | HBM alone |
| 100 µg/dl: | Standard solution (100 µl) + HBM (300 µl) |
| 200 µg/dl: | Standard solution (150 µl) + HBM (150 µl) |
| 300 µg/dl: | Standard solution (300 µl) + HBM (100 µl) |
| 400 µg/dl: | Standard solution alone |

4. Transfer 200 µl each of the supernatant of the sample, and the diluted ammonia solutions to new Eppendorf tubes.
5. Add 800 µl of a deproteinization solution and vortex them.
6. Carry out centrifugation at 8000 rpm and 4°C for 5 minutes.
7. During the centrifugation, turn on the power sources of microplate reader and personal computer, and choose Tecan i-control.
8. Put the supernatant into wells of a 96-well flat bottom plate in a volume of 60 µl each (in duplicate).
9. Add coloring test solution A to each well in a volume of 60 µl.
10. Remove a lid, set the plate on the microplate reader, and sway the plate (double click "Shaking", choose "10 sec" and "Normal", and click "Start").
11. Add coloring test solution B to each well in a volume of 30 µl.
12. Set the plate on the microplate reader, and sway the plate.
13. Add coloring test solution C to each well in a volume of 60 µl as quickly as possible.
14. Set the plate on the microplate reader, and sway the plate.
15. Carry out incubation at 37°C in a 5% CO₂ incubator for 20 minutes.
16. Cool the plate at 4°C for 10 minutes or longer to terminate the reaction.
17. Finely wipe the bottom of the plate, and set the plate on the microplate reader with adjusting the upper left corner.
18. Measure absorbance (620 or 630 nm) (double click "Measure" of "Absorbance", select the well for measurement at 620 nm in "Details", and click "Start").

### RT-PCR

1. Extract RNA by using ISOGEN II (NIPPON GENE) according to the attached protocol,
2. Measure RNA concentration with Nano Drop (registered trademark).
3. Synthesize cDNA using PrimeScript High Fidelity RT-PCR Kit.
4. Carry out PCR using Veriti 96-Well Thermal Cycler (Applied Biosystems).
5. Primer sets were as follows.

**[Table 9]**

| Primer Name | Sequence | SEQ ID NO: |
|---|---|---|
| hALB For | CCTTTGGCACAATGAAGTGGGTAACC | 1 |
| hALB Rev | CAGCAGTCAGCCATTTCACCATAGG | 2 |
| hHNF4a For | CTGCTCGGAGCCACCAAGAGATCCATG | 3 |
| hHNF4a Rev | ATCATCTGCCACGTGATGCTCTGCA | 4 |
| hOCT4 For | GACAACAATGAAAATCTTCAGGAGA | 5 |
| hOCT4 Rev | TTCTGGCGCCGGTTACAGAACCA | 6 |
| hCYP3A4 For | CCTTACATATACACACCCTTTG | 7 |
| hCYP3A4 Rev | GGTTGAAGAAGTCCTCCTAAGCT | 8 |
| hβ-actin For | TCACCACCACGGCCGAGCG | 9 |
| hβ-actin Rev | TCTCCTTCTGCATCCTGTCG | 10 |

7. Carry out electrophoresis of the RT-PCR products on 1% agarose gel.
8. Stain the gel with Gel Red, and observe it.

### Results

Photographs of the obtained iPS-HEP cells are shown in Fig. 4. The obtained cells constituted a characteristic mesh structure. The diameters of the voids were about 300 to 1000 µm, and the widths of the reticulum portions were about 20 to 200 µm. It had a thickness of about 10 to 60 µm corresponding to the width of 1 to 3 cells.

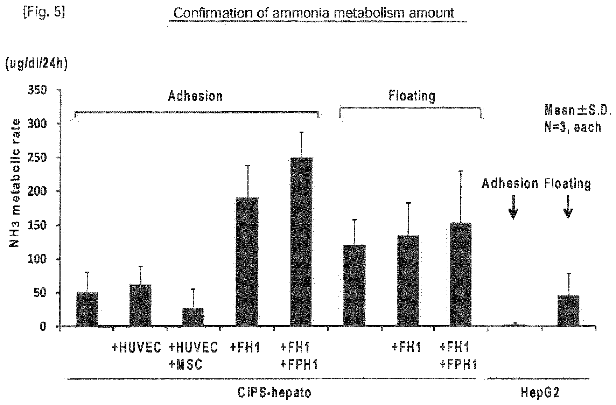

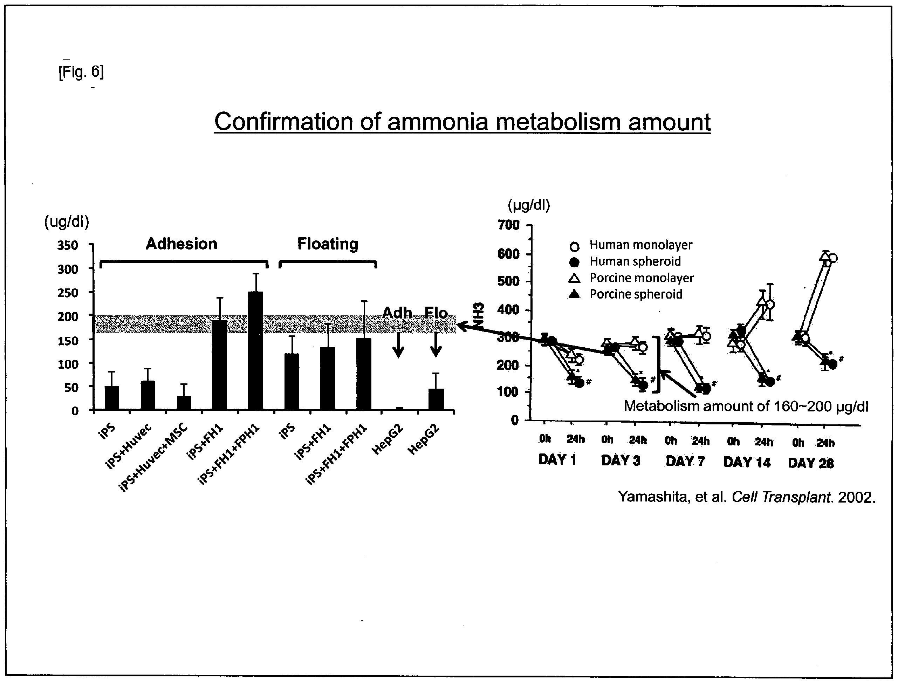

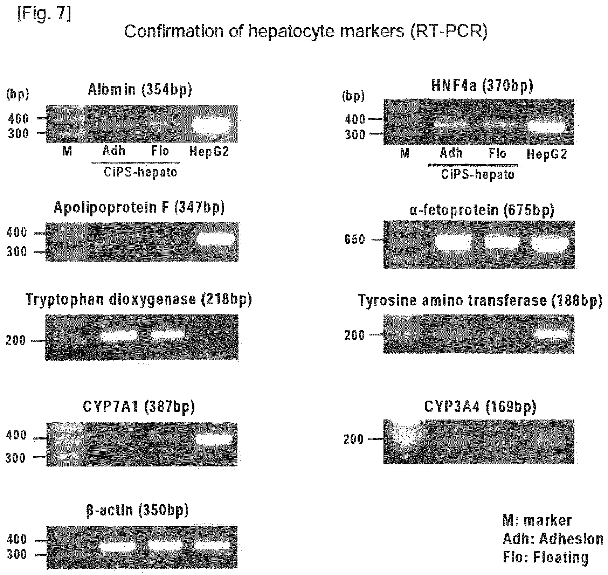

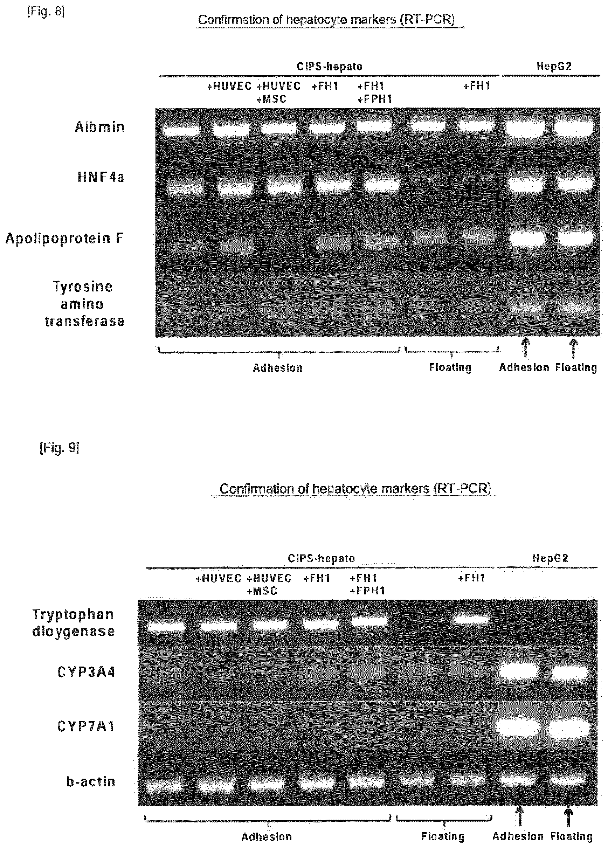

The obtained results of the ammonia metabolism test and RT-PCR are shown in Figs. 5 to 9. It was found that the iPS-HEP cells have ammonia-metabolizing function comparable to the metabolizing ability of the primarily cultured hepatocytes (160 to 200µg/dl/24h) depending on the culture conditions. Use of the compounds (FH1, FPH1) provided higher ammonia-metabolizing function-enhancing effect compared with use of feeder cells (human umbilical vein endothelial cells (HUVECs)) and mesenchymal stem cells (MSCs).

### <List of materials used in the examples>

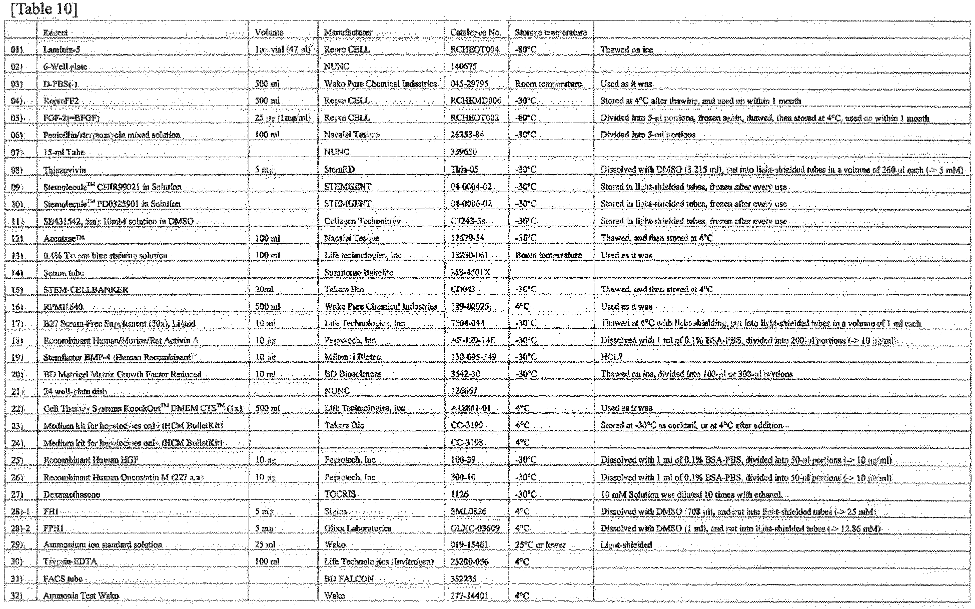

### <Reference cited in examples >

Non-patent-document 1: T. Takebe et al., Vascularized and functional human liver from an iPSC-derived organ bud transplant, Nature, 499, 481-484 (2013), doi:10.1038/nature12271, Published online 03 July 2013

### Industrial Applicability

The highly functional artificial hepatocyte induced from an iPS cell obtained by the present invention can be favorably used as a hybrid artificial liver. It can also be used for drug screening, toxicity test based on ammonia metabolism or the like as an index, and so forth.

### Sequence Listing Free Text

SEQ ID NO: 1, hALB For
SEQ ID NO: 2, hALB Rev
SEQ ID NO: 3, hHNF4a For
SEQ ID NO: 4, hHNF4a Rev
SEQ ID NO: 5, hOCT4 For
SEQ ID NO: 6, hOCT4 Rev
SEQ ID NO: 7, hCYP3A4 For
SEQ ID NO: 8, hCYP3A4 Rev
SEQ ID NO: 9, hβ-actin For
SEQ ID NO: 10, hβ-actin Rev

## Claims

1. A method for preparing an artificial hepatocyte comprising the following steps:
the differentiation induction step 1 of culturing an iPS cell in a differentiation induction medium I containing activin A,
the differentiation induction step 2 of culturing a cell obtained in the differentiation induction step 1 in a differentiation induction medium II containing bone morphogenetic protein 4 (BMP4) and fibroblast growth factor 2 (FGF2); and
the differentiation induction step 3 of culturing a cell obtained in the differentiation induction step 2 in a differentiation induction medium III containing hepatocyte growth factor (HGF), oncostatin M, dexamethasone, and N,N'-(methylenebis)(4,1-phenylene)diacetamide (FH1) and 2-N-(5-chloro-2-methylphenyl)(methylsulfonamido)-N-(2,6-difluorophenyl)acetamide (FPH1) to obtain an artificial hepatocyte.

2. The preparation method according to claim 1, wherein the iPS cell has been produced by using a Sendai virus vector.

3. The preparation method according to claim 1 or 2, wherein the differentiation induction steps 1 to 3 are performed in a serum-free and feeder-free environment.

4. The preparation method according to any one of claims 1 to 3, wherein the differentiation induction step 1 and the differentiation induction step 2 are performed for 2 to 14 days in total, and the differentiation induction step 3 is performed for 2 to 14 days.

## Patentansprüche

1. Verfahren zur Herstellung eines künstlichen Hepatocyten, umfassend die folgenden Schritte:
den Differenzierungsinduktionsschritt 1 des Kultivierens einer iPS-Zelle in einem Differenzierungsinduktionsmedium I, das Activin A enthält,
den Differenzierungsinduktionsschritt 2 des Kultivierens einer im Differenzierungsinduktionsschritt 1 erhaltenen Zelle in einem Differenzierungsinduktionsmedium II, das knochenmorphogenetisches Protein 4 (bone morphogenetic protein 4; BMP4) und Fibroblasten-Wachstumsfaktor 2 (fibroblast growth factor 2; FGF2) enthält; und
den Differenzierungsinduktionsschritt 3 des Kultivierens einer im Differenzierungsinduktionsschritt 2 erhaltenen Zelle in einem Differenzierungsinduktionsmedium III, das Hepatocytenwachstumsfaktor (hepatocyte growth factor; HGF), Oncostatin M, Dexamethason und N,N'-(Methylenbis)(4,1-phenylen)diacetamid (FH1) und 2-N-(5-chlor-2-methylphenyl)(methylsulfonamido)-N-(2,6 difluorphenyl)acetamid (FPH1) enthält, um einen künstlichen Hepatocyten zu erhalten.

2. Herstellungsverfahren nach Anspruch 1, wobei die iPS-Zelle unter Verwendung eines Sendai-Virus-Vektors hergestellt wurde.

3. Herstellungsverfahren nach Anspruch 1 oder 2, wobei die Differenzierungsinduktionsschritte 1 bis 3 in einer Serum- und Feeder-freien Umgebung durchgeführt werden.

4. Herstellungsverfahren nach einem der Ansprüche 1 bis 3, wobei der Differenzierungsinduktionsschritt 1 und der Differenzierungsinduktionsschritt 2 insgesamt 2 bis 14 Tage lang durchgeführt werden und der Differenzierungsinduktionsschritt 3 2 bis 14 Tage lang durchgeführt wird.

## Revendications

1. Méthode pour préparer un hépatocyte artificiel, comprenant les étapes suivantes :
une étape 1 d'induction de différenciation dans laquelle une cellule iPS est cultivée dans un milieu I d'induction de différenciation contenant de l'activine A,
une étape 2 d'induction de différenciation dans laquelle une cellule obtenue dans l'étape 1 d'induction de différenciation est cultivée dans un milieu II d'induction de différenciation contenant de la protéine morphogénétique osseuse 4 (BMP4) et du facteur de croissance des fibroblastes 2 (FGF2) ; et
une étape 3 d'induction de différenciation dans laquelle une cellule obtenue dans l'étape 2 d'induction de différenciation est cultivée dans un milieu III d'induction de différenciation contenant du facteur de croissance des hépatocytes (HGF), de l'oncostatine M, de la dexaméthasone, et du N,N'-(méthylènebis)(4,1-phénylène)diacétamide (FH1) et du 2-N-(5-chloro-2-méthylphényl)(méthylsulfonamido)-N-(2,6-difluorophényl)acétamide (FPH1) pour que soit obtenu un hépatocyte artificiel.

2. Méthode de préparation selon la revendication 1, dans laquelle la cellule iPS a été produite par utilisation d'un vecteur viral Sendai.

3. Méthode de préparation selon la revendication 1 ou 2, dans laquelle les étapes 1 à 3 d'induction de différenciation sont réalisées dans un environnement sans sérum et sans cellules nourricières.

4. Méthode de préparation selon l'une quelconque des revendications 1 à 3, dans laquelle l'étape 1 d'induction de différenciation et l'étape 2 d'induction de différenciation sont réalisées pendant 2 à 14 jours au total, et l'étape 3 d'induction de différenciation est réalisée pendant 2 à 14 j ours.
